(19) Europäisches Patentamt
European Patent Office
Office européen des brevets

(11) Veröffentlichungsnummer : **0 067 356**
**B1**

(12) **EUROPÄISCHE PATENTSCHRIFT**

(45) Veröffentlichungstag der Patentschrift : **30.01.85**

(51) Int. Cl.⁴ : **C 07 H 15/20**, C 07 H 15/22, A 61 K 31/70

(21) Anmeldenummer : **82104775.0**

(22) Anmeldetag : **01.06.82**

(54) **Gesättigte Aminocyclitderivate, ihre Herstellung und sie enthaltende Arzneimittel.**

(30) Priorität : **13.06.81 DE 3123520**

(43) Veröffentlichungstag der Anmeldung : **22.12.82 Patentblatt 82/51**

(45) Bekanntmachung des Hinweises auf die Patenterteilung : **30.01.85 Patentblatt 85/05**

(84) Benannte Vertragsstaaten : **AT BE CH DE FR GB IT LI SE**

(56) Entgegenhaltungen :
**FR-A- 2 375 248**
**GB-A- 2 011 397**
**GB-A- 2 060 629**
**JOURNAL OF ANTIBIOTICS, Band XXXIV, Nr. 11, 6. Juni 1981, Seiten 1429-1433**

(73) Patentinhaber : **BAYER AG**
**Konzernverwaltung RP Patentabteilung**
**D-5090 Leverkusen 1 Bayerwerk (DE)**

(72) Erfinder : **Junge, Bodo, Dr.**
**Spiekern 23**
**D-5600 Wuppertal 23 (DE)**
Erfinder : **Müller, Lutz, Dr.**
**c/o Miles Laboratories, Inc. Biotechnology R & D**
**Elkhart Indiana 46515 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 067 356 B1

Jouve, 18, rue St-Denis, 75001 Paris, France

**Beschreibung**

Die vorliegende Erfindung betrifft neue gesättigte Aminocyclitderivate, ein Verfahren zu ihrer Herstellung sowie ihre Verwendung als Arzneimittel, insbesondere als Mittel gegen Diabetes, Hyperlipämie und Adipositas.

Die erfindungsgemäßen Verbindungen haben die allgemeine Formel I

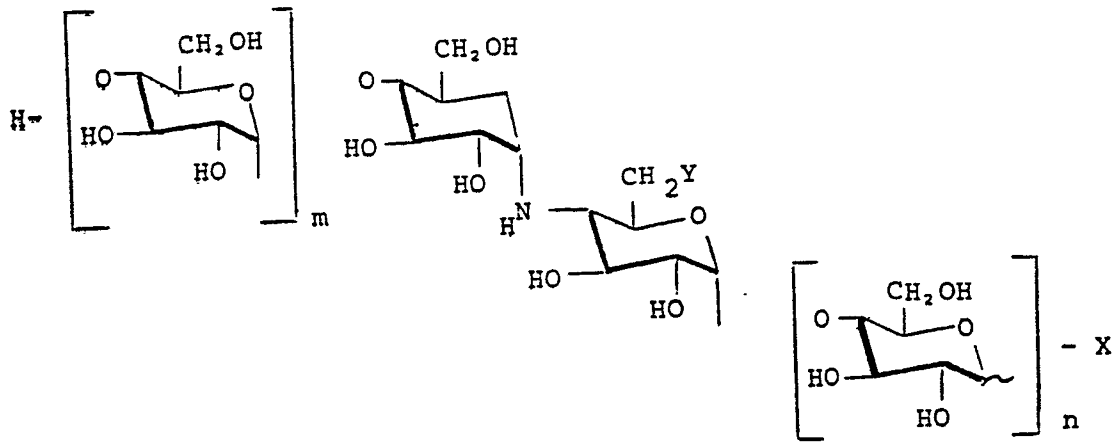

in der

m und n unabhängig voneinander eine ganze Zahl von 0 bis 8 bedeuten und die Summe von m + n einen Wert von 0 bis 8 annimmt,

X für OH, OR, SH, SR, $NH_2$, NHR oder $NRR_1$ steht, und

R und $R_1$ für gegebenenfalls substituiertes Alkyl, gesättigtes oder ungesättigtes Cycloalkyl, Aryl, Aralkyl oder Heterocyclyl stehen oder zusammen mit dem Stickstoffatom, an das sie gebunden sind, einen heterocyclischen Ring bilden, und Y für H oder OH steht.

Bevorzugt sind Verbindungen, in denen m und n Werte von 0 bis 3 annehmen, besonders bevorzugt Verbindungen, in denen m = 0 und n = 2 oder 0 ist.

R in der Bedeutung von Alkyl steht bevorzugt für geradkettiges oder verzweigtes Alkyl mit 1 bis 30, insbesondere 1 bis 18 Kohlenstoffatomen. Beispielhaft seien Methyl, Ethyl, n-Propyl, i-Propyl, n-Butyl, n-Butyl, n-Hexyl, n-Octyl, Octyl-(2), Docecyl, Lauryl, Cetyl und Stearyl genannt.

Die Alkylreste können einen oder mehrere vorzugsweise 1 bis 5, gleiche oder verschiedene Substituenten tragen. Als Substituenten seien beispielhaft aufgeführt : Hydroxy, Alkoxy mit vorzugsweise 1 bis 4 Kohlenstoffatomen, insbesondere Methoxy und Ethoxy ; Amino, Monoalkylamino und Dialkylamino mit vorzugsweise 1 bis 4 Kohlenstoffatomen je Alkylrest, insbesondere Monomethylamino, Monoethylamino, Dimethylamino und Diethylamino ; Mercapto, Alkylthio mit vorzugsweise 1 bis 4 Kohlenstoffatomen, insbesondere Methylthio und Ethylthio ; Halogen, vorzugsweise Fluor, Chlor und Brom ; Alkylcarbonyl mit vorzugsweise 1 bis 4 Kohlenstoffatomen im Alkylrest ; Carboxy, Nitro, Cyan, die Aldehydfunktion und die Sulfonsäuregruppe.

Es sei erwähnt, daß für R in der Bedeutung als substituierter Alkylrest auch die von Zuckerderivaten wie Polyalkoholen oder Zuckersäuren abgeleiteten Reste besonderes Interesse im Rahmen der vorliegenden Anmeldung verdienen.

R in der Bedeutung von Cycloalkyl steht vorzugsweise für einen gesättigten oder ungesättigten carbocyclischen Rest mit vorzugsweise 3 bis 7 Kohlenstoffatomen, der substituiert sein kann, wobei als Substituenten die bei den offenkettigen Kohlenwasserstoffresten genannten Gruppen und Atome in Betracht kommen. Insbesondere steht R in der Bedeutung von Cycloalkyl auch für einen gesättigten oder ungesättigten Cyclitrest.

R in der Bedeutung von Aryl oder Aralkyl steht vorzugsweise für aromatische Reste mit 6 bis 10 Kohlenstoffatomen im Arylteil, insbesondere Phenyl, die substituiert sein können.

Die Arylreste können einen oder mehrere, vorzugsweise 1 bis 3 gleiche oder verschiedene Substituenten tragen. Als Substituenten seien beispielhaft aufgeführt : Alkyl mit 1 bis 10 Kohlenstoffatomen, die ihrerseits wieder beispielsweise durch Chlor, Nitro oder Cyan substituiert sein können ; gegebenenfalls substituierte Alkylreste mit 1 bis 10 Kohlenstoffatomen ; Hydroxy, Alkoxy mit vorzugsweise 1 bis 4 Kohlenstoffatomen ; Amino, Monoalkyl- und Dialkylamino mit vorzugsweise 1 bis 4 Kohlenstoffatomen je Alkylrest ; Mercapto, Alkylthio mit vorzugsweise 1 bis 4 Kohlenstoffatomen ; Carboxy, Carbalkoxy mit vorzugsweise 1 bis 4 Kohlenstoffatomen.

Die Sulfonsäuregruppe, Alkylsulfonyl mit vorzugsweise bis 4 Kohlenstoffatomen, Arylsulfonyl, vorzugsweise Phenylsulfonyl ; Aminosulfonyl-, Alkylamino- und Dialkylaminosulfonyl mit 1 bis 4 Kohlenstoffatomen je Alkylgruppe, vorzugsweise Methyl- und Dimethylaminosulfonyl ; Nitro, Cyan oder die Aldehydgruppe ; Alkylcarbonylamino mit vorzugsweise 1 bis 4 Kohlenstoffatomen ; Alkylcarbonyl mit 1 bis 4 Kohlenstoffatomen, Benzoyl, Benzylcarbonyl und Phenylethylcarbonyl, wobei die zuletzt genannten

Alkyl, Phenyl, Benzyl und Phenylethylreste ihrerseits wieder beispielsweise durch Chlor, Nitro oder Hydroxy substituiert sein können. Wenn R für Aralkyl steht, so ist der entsprechende Alkylteil vorzugsweise eine gesättigte oder ungesättigte Kette mit bis zu 6 C-Atomen, z. B. Benzyl, Phenetyl, Phenylpropyl oder Cinnamyl.

Bevorzugte heterocyclische Reste R sind von heteroparaffinischen, heteroaromatischen oder heteroolefinischen 5- oder 6-gliedrigen Ringen mit vorzugsweise 1 bis 3 gleichen oder verschiedenen Heteroatomen abgeleitet. Als Heteroatome stehen Sauerstoff, Schwefel oder Stickstoff. Diese Ringsysteme können weitere Substituenten wie beispielsweise Hydroxy-, Amino- oder $C_1$-$C_4$-Alkylgruppen tragen oder an sie können Benzolkerne oder weitere vorzugsweise 6-gliedrige heterocyclische Ringe der genannten Art anelliert sein.

Dabei erfolgt die Bindung des Heterocyclischen Restes R über ein Kohlenstoffatom des heterocyclischen Systems oder des anellierten Benzolkerns. Unter heterocyclischen Resten werden auch solche verstanden, die über eine außerhalb des Ringes stehende $—CH_2$-Brücke gebunden sind wie beispielsweise der Furfurylrest.

$R_1$ steht vorzugsweise für einen geradkettigen oder verzweigten Alkylrest mit 1 bis 6 Kohlenstoffatomen, den Benzyl- oder Phenylrest, wobei die genannten Reste vorzugsweise durch Alkoxy mit 1 bis 4 Kohlenstoffatomen, Amino, $C_1$-$C_4$-Monoalkyl- und $C_1$-$C_4$-Dialkylamino, Nitro, Halogen, Cyan, Hydroxy, Nitro, Mercapto, $C_1$-$C_4$-Thioalkyl, die Carboxy- oder Sulfonsäuregruppe und im Falle, daß $R_1$ Phenyl bedeutet auch durch $C_1$-$C_4$-Alkyl substituiert sein können.

Wie oben ausgeführt, können R und $R_1$ auch zusammen unter Einschluß des Stickstoffatomes an das sie gebunden sind, einen heterocyclischen Ring bilden.

Dieser Ring kann gesättigt oder ungesättigt sein sowie 1 bis 3 weitere, vorzugsweise 1 Sauerstoff-, Schwefel- oder Stickstoffatom und als Heterogruppen vorzugsweise eine $SO_2$- oder N-Alkyl-Gruppe enthalten, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder. Der 6-gliedrige heterocyclische Ring enthält vorzugsweise das Heteroatom oder die Heterogruppe in para-Stellung zum Stickstoffatom. Als Beispiele für den heterocyclischen Ring seien Pyrrolidin, Piperidin, Piperazin, Hexamethylenimin, Morpholin und n-Methylpiperazin genannt.

Daß die erfindungsgemäßen gesättigten Aminocyclitderivate der Formel I potente Inhibitoren für α-Glykosidhydrolasen insbesondere Disaccharidasen des Verdauungstrakts sind ist überraschend. Zwar ist aus einer Reihe von Druckschriften, z. B. DE-OS 2 347 782 : DE-OS 2 614 393 : US 4 175 123 : US 4 197 292 : DOS 2 855 409 : Naturwissenschaften *64,* 535 (1977) bekannt, daß zu den Verbindungen der Formel I analoge ungesättigte Verbindungen mit einer Doppelbindung im Cyclitteil des Moleküls wirksame Inhibitoren für α-Glykosidhydrolasen sind. Nach der « transition state analogue »-Theorie für Enzyminhibitoren (Lindquist, R. N. : Medicinal Chemistry, Vol. 11 (V), p. 24 (Ariens, E. J. ed.), New York — San Francisco — London : Academic Press 1975) mußte man aber annehmen, daß die Doppelbindung in diesen Verbindungen ein für die Wirkung essentielles Strukturmerkmal ist. Nur die ungesättigte Cycliteinheit ähnelt in ihrer Halbsesselkonformation nämlich dem bei der enzymatischen Reaktion aus dem Substrat (Saccharose, Maltose, Stärke) gebildeten Glucosylkation in Halbsesselkonformation.

Es sind aus der GB-A 20 60 629 in Verbindung mit « The Journal of Antibiotics », Band 34, Seiten 1429-1433 auch Verbindungen bekannt, die gesättigt sind und eine, zum Vergleich zu den erfindungsgemäßen Verbindungen, zusätzliche OH-Gruppe im Cyclitteil aufweisen. Diese Verbindungen weisen jedoch eine deutlich schwächere Inhibitionswirkung auf als die erfindungsgemäßen Verbindungen.

Um so überraschender ist es, daß auch die erfindungsgemäßen gesättigten Aminocyclitderivate mit einer α-D-gluco-Konfiguration im Cyclitteil eine ähnlich starke inhibitorische Wirkung aufweisen, wie die ungesättigten Verbindungen.

Aufgrund ihrer enzyminhibitorischen Wirksamkeit lassen sich die erfindungsgemäßen Verbindungen zur Verzögerung oder Blockierung der Kohlenhydratverdauung bei Mensch und Tier einsetzen. Sie sind daher wertvolle Mittel zur Behandlung von Stoffwechselkrankheiten wie Diabetes, Hyperlipämie und Adipositas und bereichern somit den Arzneimittelschatz.

Weiterhin wurde gefunden, daß man die erfindungsgemäßen Verbindungen der Formel I durch Hydrierung aus den ungesättigten Verbindungen der Formel II

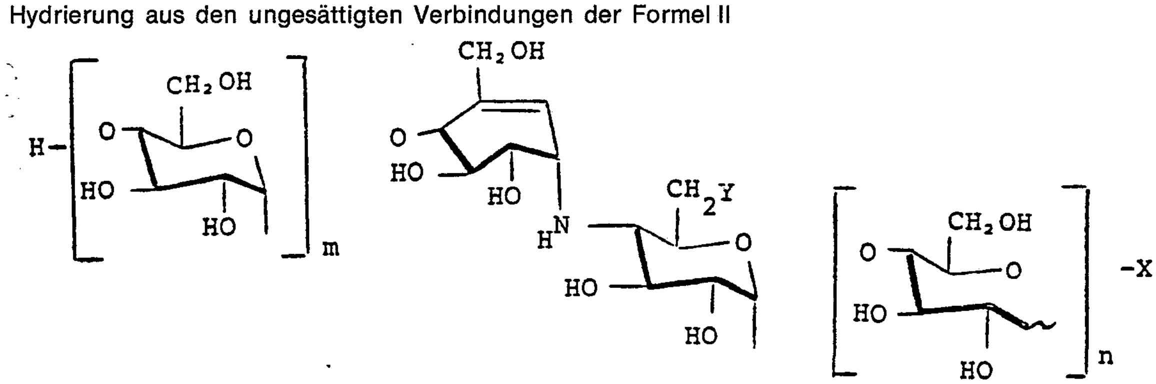

(II)

erhält, die in den oben zitierten Patentanmeldungen und Publikationen beschrieben werden. Die Hydrierung kann katalytisch oder mit geeigneten Mikroorganismen erfolgen.

Bei der katalytischen Hydrierung, vorzugsweise mit Übergangsmetallkatalysatoren, erhält man die Verbindungen der Formel I in der Regel nur im Gemisch mit anderen Hydrogenolyse- und Hydrierungsprodukten. Insbesondere spielen als Nebenreaktionen die hydrogenolytische Spaltung der C—N-Bindung und der C—O-Bindungen in Allylstellung zur Doppelbindung eine Rolle und die Absättigung der Doppelbindung zu gesättigten Verbindungen mit L-ido-Konfiguration im Cyclitteil. Daher muß man die Verbindungen der Formel I in der Regel durch chromatographische Trennverfahren aus dem Hydrierungsgemisch isolieren.

Als Katalysatoren für die Hydrierung werden die üblichen Übergangsmetallkatalysatoren wie Pt, Pd/C oder Ni verwendet. Als Lösungsmittel werden vorzugsweise Wasser oder Wasser/Alkoholgemische verwendet. Bevorzugte Alkohole sind wassermischbar, z. B. Methanol und Ethanol. Die Hydrierung wird bevorzugt bei Raumtemperatur und Drucken zwischen 1 und 100 Atmosphären, bevorzugt zwischen 1 und 3 Atmosphären $H_2$-Druck durchgeführt.

Das folgende Formelschema soll das Herstellungsverfahren erläutern :

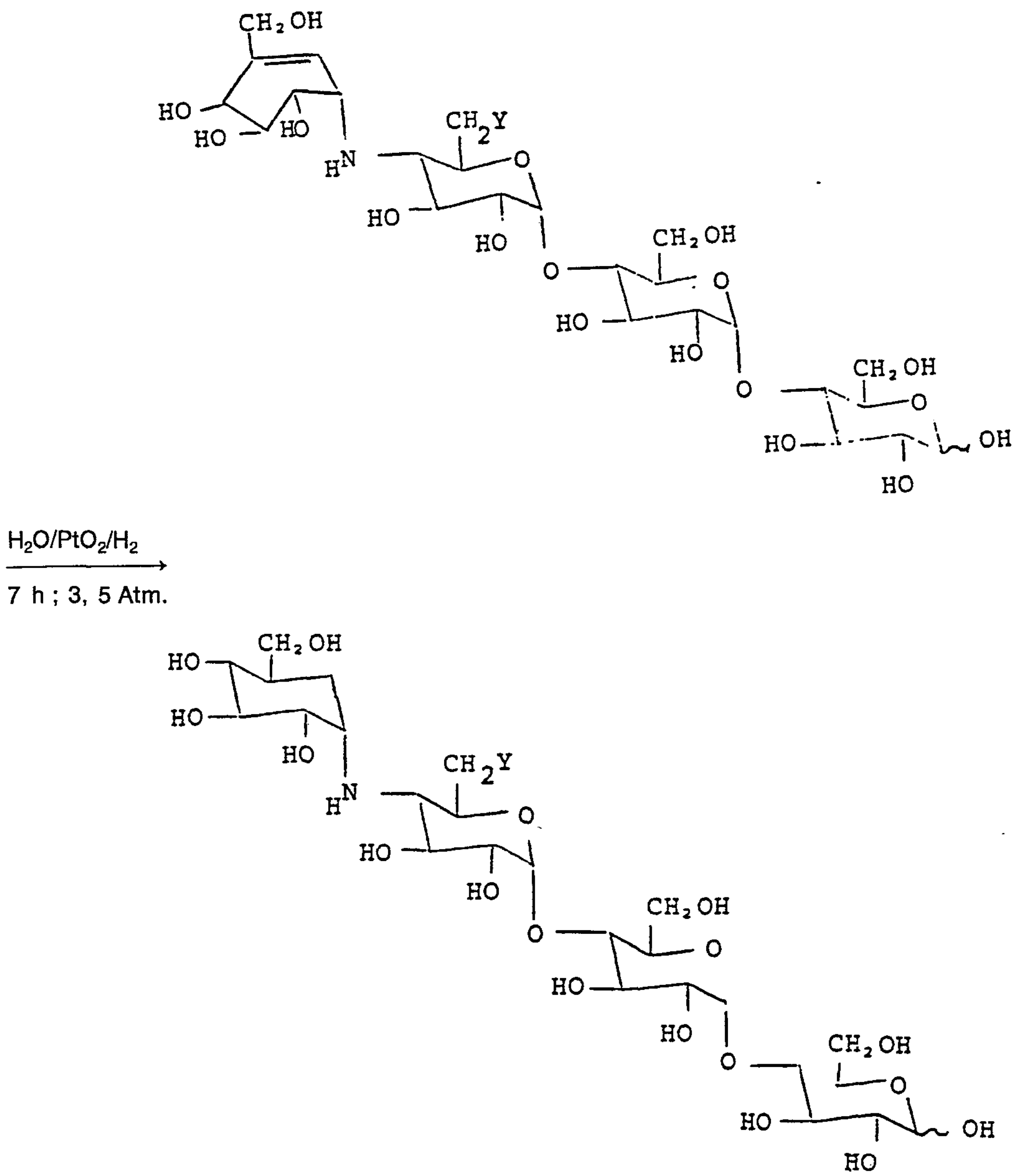


Es ist bekannt, daß bei Tieren und menschen nach Aufnahme von kohlenhydrathaltigen Nahrungs- und Genußmitteln (z. B. Getreide-, Kartoffelstärke, Obst, Fruchtsaft, Bier, Schokolade) Hyperglykämien auftreten, die infolge eines raschen Abbaus der Kohlenhydrate durch Glykosidhydrolasen (z. B. Speichel- und Pankreasamylasen, Maltasen, Saccharasen) nach folgendem Schema

Stärke bzw. Glykogen $\xrightarrow{\text{Amylase}}$ Maltose $\xrightarrow{\text{Maltase}}$ Glucose Saccharose $\xrightarrow{\text{Saccharase}}$ Glucose + Fruktose

bewirkt werden. Diese Hyperglykämien sind bei Diabetikern besonders stark und anhaltend ausgeprägt. Bei Adipösen bewirkt die alimentäre Hyperglykämie oftmals eine besonders starke Sekretion von Insulin, das seinerseits zu vermehrtem Fettaufbau und vermindertem Fettabbau führt. Im Anschluß an derartige Hyperglykämien tritt bei stoffwechselgesunden und adipösen Personen infolge der Insulinsekretion häufig eine Hypoglykämie auf. Bekannt ist, daß sowohl Hypoglykämien als auch im Magen verweilender Speisebrei die Produktion von Magensaft fördern, der seinerseits die Entstehung einer Gastritis, eines Ulcus ventriculi oder duodeni auslöst oder begünstigt.

Die erfindungsgemäßen Inhibitoren der Glykosidhydrolasen können die alimentäre Hyperglykämie, Hyperinsulinämie, Hypoglykämie nach Belastung mit Weizenstärke oder Saccharose oder Maltose erheblich vermindern und die Magenpassage dieser genannten Kohlenhydrate beschleunigen.

Ferner wird die Konversion von Kohlenhydraten in Lipide des Fettgewebe und der Einbau von alimentärem Fett in die Fettgewebedepots vermindert bzw. verzögert.

Ferner ist bekannt, daß in der Mundhöhle Kohlenhydrate, besonders Saccharose, durch Mikroorganismus gespalten werden und dadurch die Kariesbildung gefördert wird.

Inhibitoren der Glykosidhydrolasen eignen sich deshalb als Therapeuticum für folgende Indikationen :

Adipositas, Hyperlipämie (Atherosklerose), Diabetes, Prädiabetes, Gastritis, Ulcus ventriculi, Ulcus duodeni und Karies.

Die erfindungsgemäßen Verbindungen werden zweckmäßig in einer Menge von 1-10 000 SIE/kg ein- oder mehrmals täglich vor und/oder wöhrend und/oder nach den Mahlzeiten bzw. Getränken p. os. dosiert.

## Zubereitungsformen

Tablette, Dragee, Kapsel, Lösung, Suspension, Granulat, Kaugummi, Zahnpasta und als Zusatz zu kohlenhydrathaltigen Lebens- und/oder Genußmitteln.

Vorteilhaft sind auch Kombinationen der erfindungsgemäßen Inhibitoren mit den bekannten oralen Antidiabetica (β-cytotrope Sulfonylharnstoffderivate und/oder blutzuckerwirksame Biguanide).

## Beispiel 1

(Verbindung der Formel I mit $m = 0$, $n = 2$, $Y = H$ und $X = OH$).

5 g Acarbose (Verbindung der Formel II mit $m = 0$, $n = 2$, $Y = H$ und $X = OH$) wurden in 200 ml Wasser mit 2,5 g $PtO_2$ als Katalysator 7 Stunden bei 3,5 Atmosphären $H_2$-Druck bei Raumtemperatur hydriert. Anschließend wurde der Katalysator abfiltriert, die Lösung am Rotationsverdampfer eingeengt und auf eine mit Dowex® 50 W-X 4 ($H^{\oplus}$-Form) gefüllte Säule (40 × 2,4 cm) aufgetragen. Zunächst wurden die nicht basischen Hydrogenolyseprodukte mit Wasser eluiert. Es wurden Fraktionen von ca. 7 ml abgenommen. Ab Fraktion 70 wurden die basischen Hydrogenolyse- und Hydrierungsprodukte mit 0,025 n HCl eluiert. Die Fraktionen 515-536 wurden vereinigt, mit basischem Austauscher (Amberlite® IRA 410 ; $OH^{\ominus}$-Form) neutralisiert und am Rotationsverdampfer eingeengt. Es wurden 100 mg der Verbindung der Formel I mit $m = 0$, $n = 2$ und $X = OH$ erhalten.

Die Substanz wurde durch ein Protonenresonanzspektrum bei 250 MHz in $D_2O$ charakterisiert.

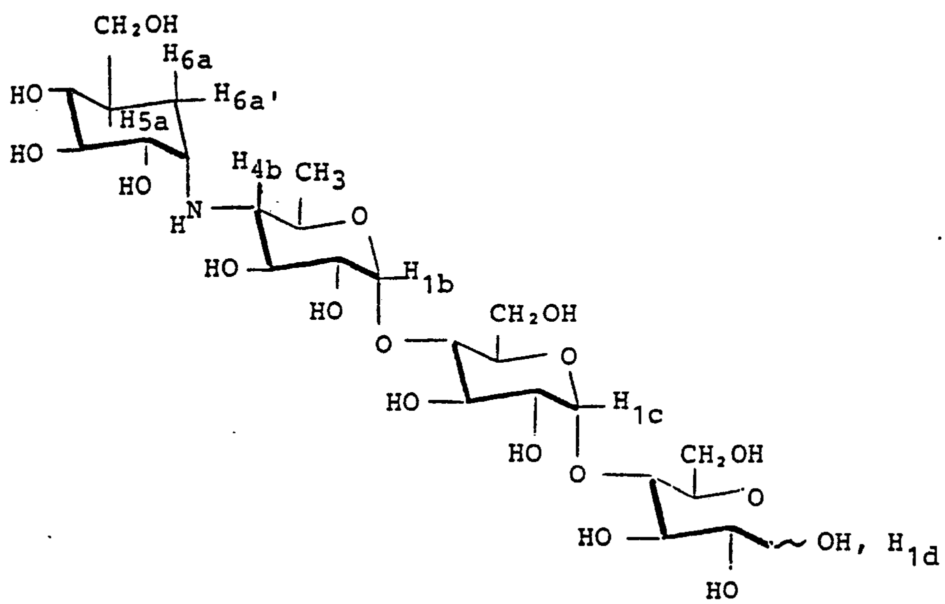

Folgende Shiftwerte lassen sich dem Spektrum entnehmen (<u>H</u>OD/bei δ = 4,67 ppm) :

| Proton | δ (ppm) | Signalform |
|---|---|---|
| $H_{6a}$ | 1,23 | Dublettiertes (J ~ 3 Hz) Triplett (J ~ 11-14 Hz) |
| $H_{6a'}$ | 1,77 | Triplettiertes (~ 3 H) Dublett (J ~ 14 Hz) |
| $H_{5a}$ | 1,65 | breites Signal |
| $H_{4b}$ | 2,26 | Triplett (J ~ 10 Hz) |
| $H_{1d}(\beta)$ | 4,50 } β/α-Verhältnis 2 : 1 | Dublett (J = 8 Hz) |
| $H_{1d}(\alpha)$ | 5,08 | Dublett (J = 3,5 Hz) |
| $H_{1c}$ | | zwei Dubletts (J ~ 3-4 Hz) bei |
| $H_{1b}$ | | 5,16 und 5,25 |
| $CH_3$-Gruppe | 1,16 | Dublett (J = 6,3 Hz) |

Beispiel 2

(Verbindung der Formel I mit m = 0, n = 0, Y = H und X = $OCH_3$).

5 g der Verbindung der Formel II mit m = 0, n = 0 und X = $OCH_3$ (α/β-Gemisch) wurden in 250 ml Wasser mit 2,5 $PtO_2$ als Katalysator 7 Stunden bei 3,5 Atmosphären $H_2$-Druck in der Schüttelbirne bei Raumtemperatur hydriert. Der Katalysator wurde abgesaugt und die Lösung im Vakuum eingeengt. Der wäßrige Rückstand wurde auf eine mit Amberlite IR 120 ($H^{\oplus}$-Form) gefüllte Säule gegeben. Die Säule wurde zuerst mit Wasser und dann mit 4 %igem wäßrigem Ammoniak eluiert. Das wäßrige Eluat wurde verworfen, das ammoniakalische Eluat wurde am Rotationsverdampfer zur Trockne eingeengt. Der Rückstand wurde auf zwei hintereinander-geschalteten Merck-Kieselgel-Fertigsäulen der Größe C Chromatographiert. Als Fließmittel wurden Essigsäure/MeOH/$H_2O$/25 %iges $NH_3$ im Verhältnis 100/60/20/2 verwendet. Es wurden Fraktionen von ca. 5 ml aufgefangen. Die Fraktionen 163-200 lieferten 276 mg noch nicht ganz reine Verbindung der Formel I mit m = 0, n = 0 und X = $OCH_3$. Zur Nachreinigung wurde über eine Merck-Kieselgel-Fertigsäule der Größe B chromatographiert. Als Fließmittel wurde Essigester/MeOH/$H_2O$/25%iges $NH_3$ im Verhältnis 100/50/10/1 verwendet. Es wurden 102 g der Verbindung der Formel I mit m = 0, n = 0 und X = $OCH_3$ als α/β-Gemisch erhalten.

Die Substanz wurde durch eine Protonenresonanzspektrum bei 250 MHz in $CD_3OD$ charakterisiert.

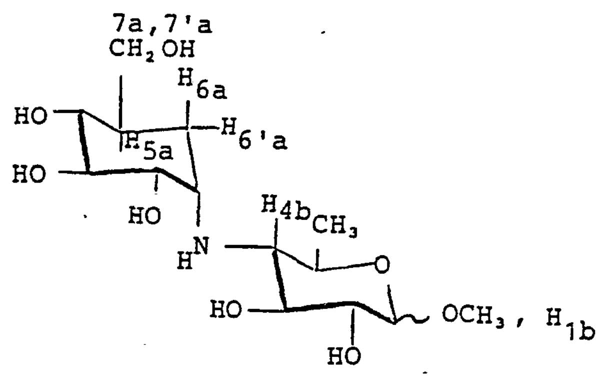

Dem Spektrum lassen sich folgende Shiftwerte ($CD_3O$<u>H</u> bei δ = 4,78 ppm) entnehmen :

| Proton | δ (ppm) | Signalform |
|---|---|---|
| $CH_3$-Gruppe | 1,20 (α) | Dublett (J = 6 Hz) |
| | 1,25 (β) | Dublett (J = 6 Hz) |
| $H_{6a}$ | ~ 1,22 | wird durch die Methylgruppe weitgehend verdeckt |
| $H_{6'a}$ | 1,80 | Überlagerung |
| $H_{5a}$ | ~ 1,77 | Überlagerung |

(Fortsetzung)

| Proton | δ (ppm) | Signalform |
|---|---|---|
| $H_{4b}$ | 2,19 (β) | Triplett (J ~ 10 Hz) |
| | 2,17 (α) | Triplett (J ~ 10 Hz) |
| $—OCH_3$ | 3,30 (α) | Singlett |
| | 3,42 (β) | Singlett |
| $H_7$ u. $H_{7'}$ | ~ 3,56 u. 3,62 | AB-System (J = 10,5 Hz) mit zusätzl. Aufspaltung ($J_1$ = 5,5 Hz u. $J_2$ = 4 Hz) |
| $H_{1b}$ (β) | 4,04 } β/α-Verhält- | Dublett (J = 7,5 Hz) |
| $H_{1b}$(α) | 4,53 } nis 3 : 1 | Dublett (J = 3,5 Hz) |

Saccharasetest

Eine Saccharase-Inhibitor-Einheit (SIE) ist definiert als die Menge Inhibitor, die zwei Saccharaseeinheiten zu 50 % inhibiert. Eine Saccharaseeinheit (SE) ist die Menge an Enzym, die in einer Minute unter den unten agegebenen Testbedingungen 1 μMol Saccharose in Glucose und Fructose spaltet. Die μMol gebildete Glucose werden mit Hilfe der Glucoseoxidaserreaktion quantitativ bestimmt unter Bedingungen, bei denen eine weitere Saccharosespaltung durch die Saccharase nicht mehr stattfindet. Zur Durchführung des Testes werden 0,05 ml einer auf 0,12 SE eingestellten Saccharaselösung[1)] mit 0-20 μg Inhibitor oder 0-20 μl der zu testenden Lösung versetzt und mit 0,1 m Natriummaleinatpuffer pH 6,0 oder 0,1 ml aufgefüllt. Es wird 10 Minuten bei 35 °C äquilibriert und dann mit 0,μ ml einer auf 35 °C vorgewärmten 0,05 m Saccharoselösung in 0,1 m Natriummaleinatpuffer pH 6,0 versetzt. Man inkubiert 20 Minuten bei 35 °C und stoppt die Saccharasereaktion durch Zugabe von 1 ml Glucoseoxidasereagens[2)] ab und inkubiert weitere 30 Minuten bei 35 °C. Danach wird 1 ml 50 % $H_2SO_4$ zugesetzt und bei 545 nm gegen einen entsprechenden Leerwert gemessen. Zur Auswertung wird die prozentuale Hemmung der eingesetzten Saccharase berechnet und aus dem 50 % Hemmpunk mi Hilfe einer Glucoseeichkurve auf SIE/g bzw. SIE/Liter umgerechnet.

Wirksamkeit im Saccharasehemmtest in vitro

Beispiel 1

| | SIE/g |
|---|---|
| Substanz der Formel I mit m = 0, n = 2, Y = H und X = OH | 59 829 |

Vergleich

| | |
|---|---|
| Substanz der Formel II mit m = 0, n = 2, Y = H und X = OH (Acarbose) | 77 700 |

Beispiel 2

| | |
|---|---|
| Substanz der Formel I mit m = 0, n = 0, Y = H und X = $OCH_3$ (α/β-Gemisch 1 : 3) | 38 850 |

Vergleich

| | |
|---|---|
| Substanz der Formel II mit m = 0, n = 0, Y = H und X = $OCH_3$ (α/β-Gemisch 1 : 9) | 38 850 |

[1)] Solubilisierte Saccharase aus Schweinedünndarmmucosa nach B. Borgström, A. Dahlquist, Acta Chem. Scand. *12*, (1958), Seite 1997. Mit 0,1 m Natriummaleinatpuffer pH 6,0 auf entsprechenden Se-Gehalt verdünnt.

[2)] Das Glucoseoxidasereagens wird durch Lösen von 2 mg Glucoseoxidase (Fa. Boehringer Nr. 15423) in 100 ml 0,565 m Tris-HCl-Puffer pH 7,0 und anschließenden Zusatz von 1 ml Detergenslösung (2 g Triton® × 100 + 8 g 95 % Ethanol p. a.), 1 ml Dianisidinlösung (260 mg o-Dianisidin · 2 HCl in 20 ml $H_2O$) und 0,5 ml 0,1 %iger wäßriger Peroxidaselösung Fa. Boehringer Nr. 15302) hergestellt.

**Ansprüche**

1. Verbindungen der Formel

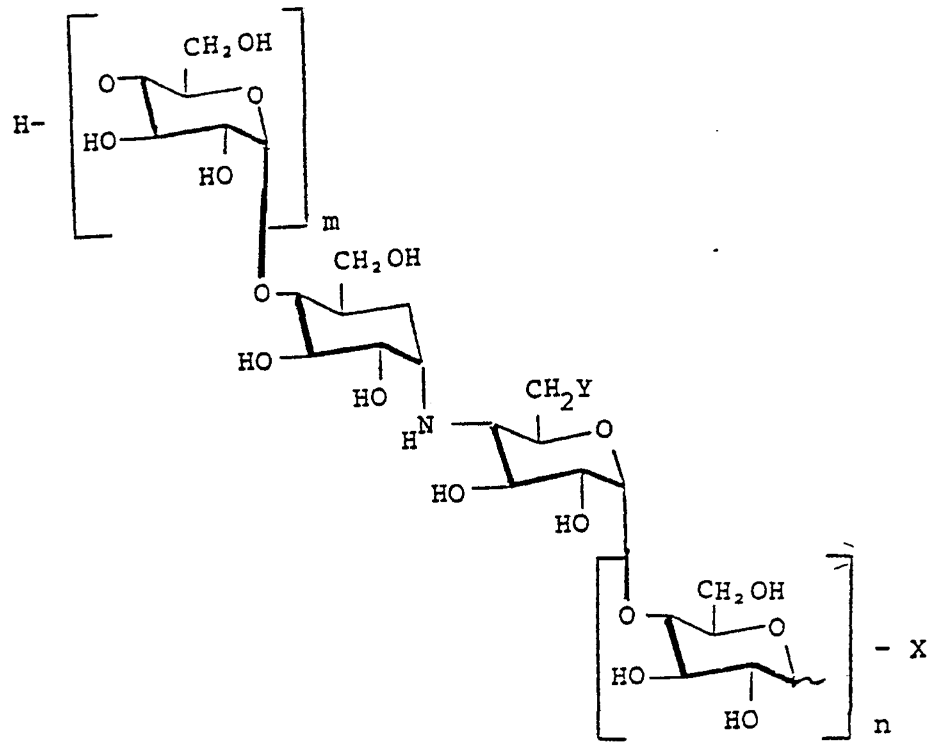

in der
m und n unabhängig voneinander eine ganze Zahl von 0 bis 8 bedeuten und die Summe von m + n einen Wert von 0 bis 8 annimmt,
X für OH, OR, SH, SR, $NH_2$, NHR oder $NRR_1$ steht, und
R und $R_1$ für gegebenenfalls substituiertes Alkyl, Cycloalkyl, Aryl, Aralkyl oder Heterocyclyl stehen oder zusammen mit dem Stickstoffatom, an das sie gebunden sind, einen heterocyclischen Ring bilden, und Y für H oder OH steht.

2. Verbindungen nach Anspruch 1, dadurch gekennzeichnet, daß m und n eine Zahl von 0 bis 3 darstellen.

3. Verbindungen nach Anspruch 1 dadurch gekennzeichnet, daß, m = 0 und n = 0 oder 2 ist.

4. Verbindungen nach den Ansprüchen 1-3, dadurch gekennzeichnet, daß X die Bedeutung OH hat.

5. Verbindungen nach den Ansprüchen 1-4, dadurch gekennzeichnet, daß Y die Bedeutung H hat.

6. Verfahren zur Herstellung der Verbindungen der allgemeinen Formel

(Siehe Figur Seite 9 f.)

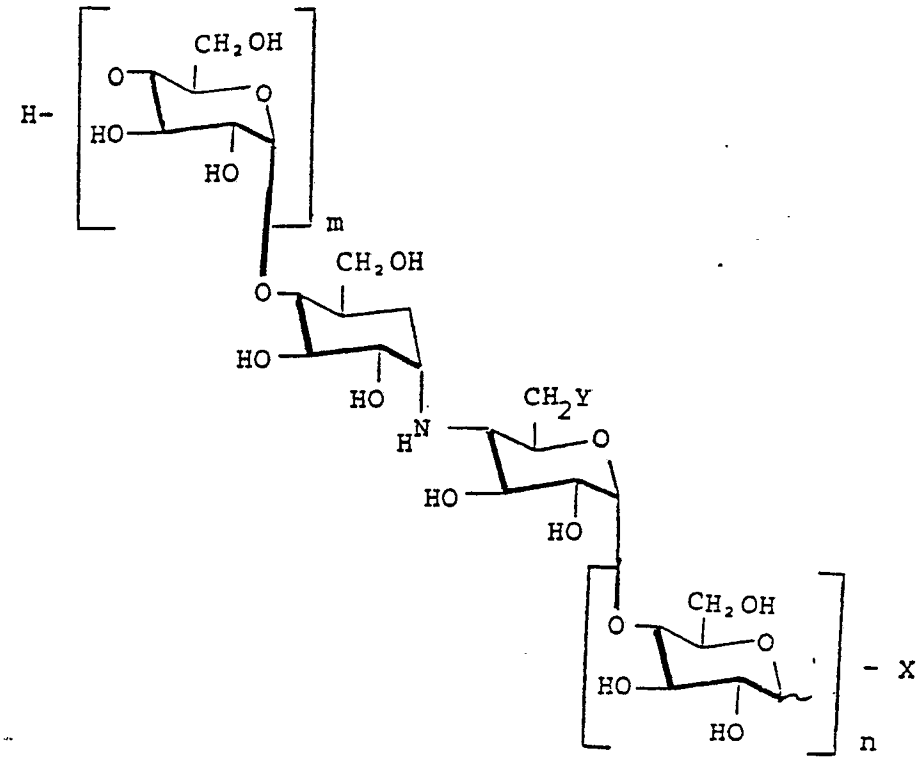

(I)

in der

m und n unahängig voneinander eine ganze Zahl von 0 bis 8 bedeuten und die Summe von m + n einen Wert von 0 bis 8 annimmt.

X für OH, OR, SH, SR, $NH_2$, NHR oder $NRR_1$ steht,

Y für H oder OH steht und

R und $R_1$ für gegebenenfalls substituiertes Alkyl, Cycloalkyl, Aryl, Aralkyl oder Heterocyclyl steht oder zusammen mit dem Stickstoffatom, an das sie gebunden sind, einen heterocyclischen Ring bilden, dadurch gekennzeichnet, daß man Verbindungen der allgemeinen Formel II

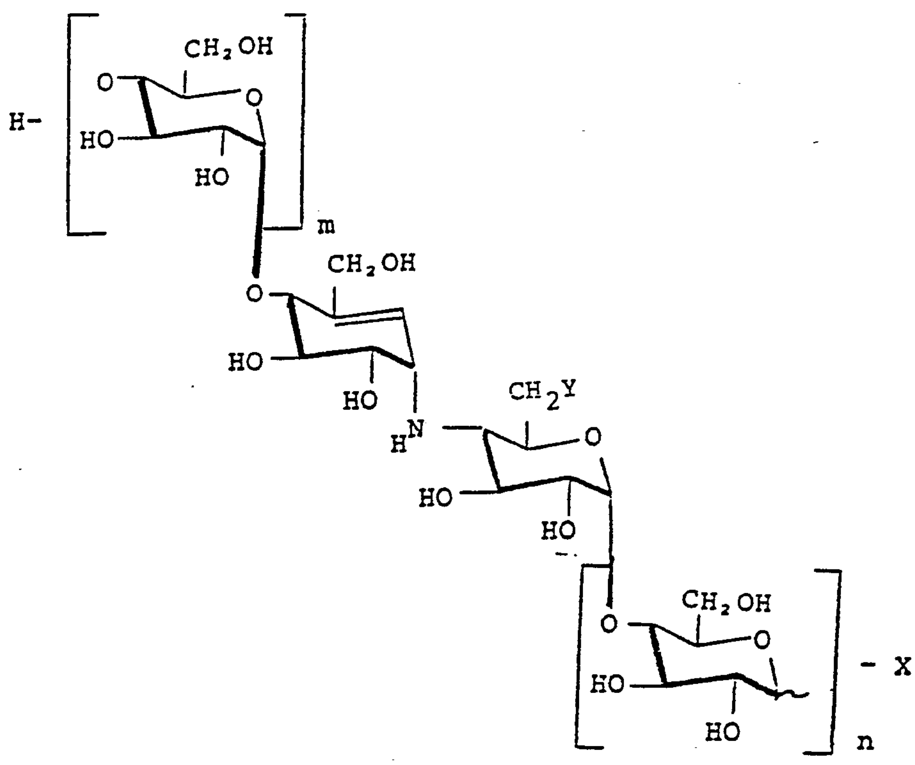

(II)

in der m, n und X die obengenannte Bedeutung haben, hydriert.

7. Verfahren nach Anspruch 6, dadurch gekennzeichnet, daß die Hydrierung katalytisch bei Raumtemperatur und Drucken von 1 bis 100 Atmosphären durchgeführt wird.

8. Arzneimittel enthaltend mindestens eine Verbindung gemäß den Ansprüchen 1-5.

## Claims

1. Compounds of the formula

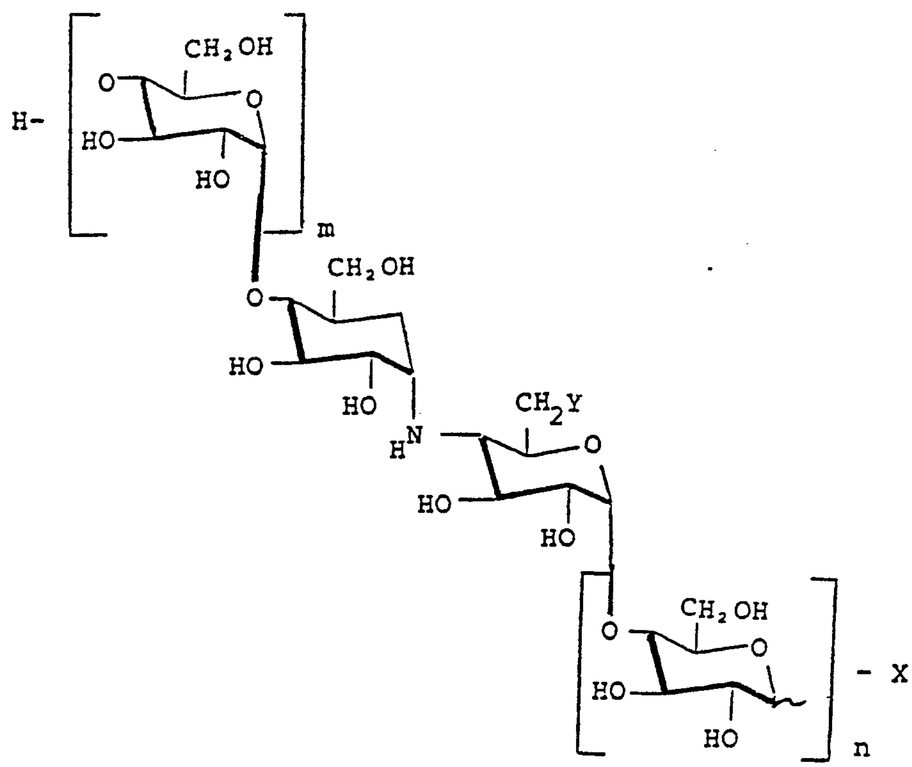

in which

m and n independently of one another denote an integer from 0 to 8 and the sum of m + n has a value of 0 to 8,

X represents OH, OR, SH, SR, $NH_2$, NHR or $NRR_1$, and

R and $R_1$ represent optionally substituted alkyl, cycloalkyl, aryl, aralkyl or a heterocyclic radical or together with the nitrogen atom to which they are bonded form a heterocyclic ring, and Y represents H or OH.

2. Compounds according to Claim 1, characterised in that m and n represent a number from 0 to 3.

3. Compounds according to Claim 1, characterised in that m = 0 and n = 0 or 2.

4. Compounds according to Claims 1-3, characterised in that X has the meaning OH.

5. Compounds according to Claims 1-4, characterised in that Y has the meaning H.

6. Process for the preparation of compounds of the general formula

(See figure page 11)

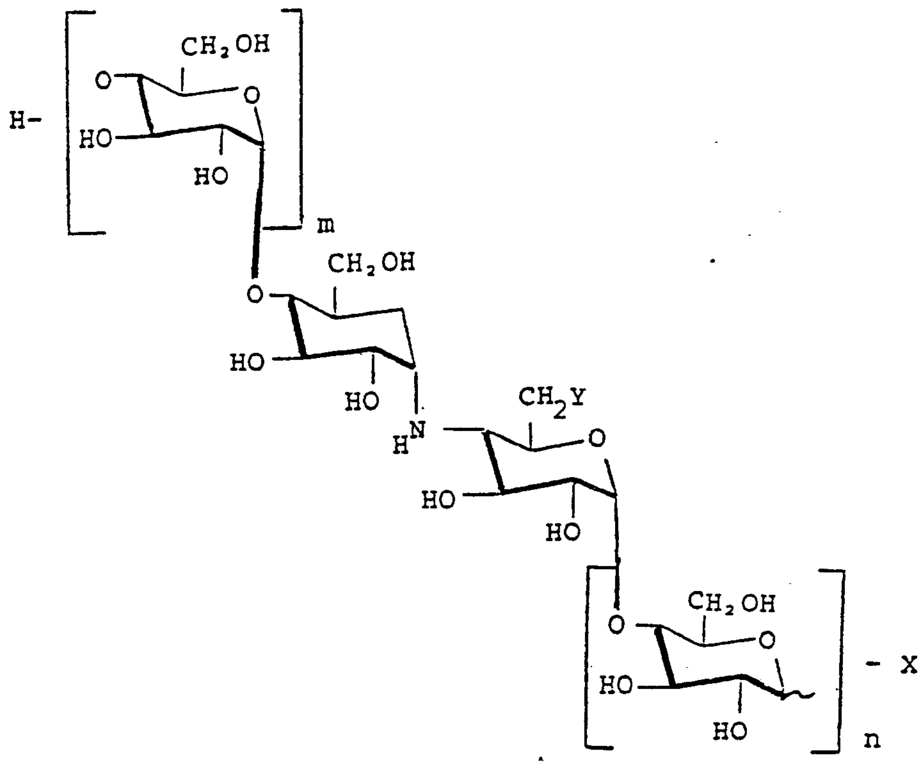

(I)

in which

m and n independently of one another denote an integer from 0 to 8 and the sum of m + n has a value of 0 to 8.

X represents OH, OR, SH, SR, $NH_2$, NHR or $NRR_1$,

Y represents H or OH, and

R and $R_1$ represent optionally substituted alkyl, cycloalkyl, aryl, aralkyl or a heterocyclic radical or together with the nitrogen atom to which they are bonded form a heterocyclic ring, characterised in that compounds of the general formula II

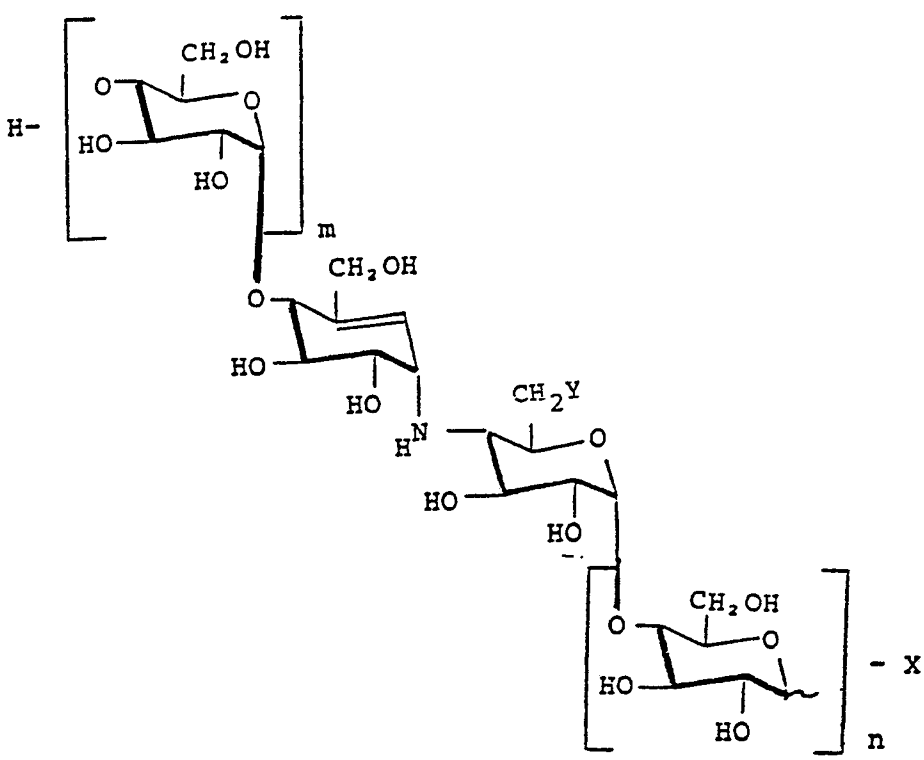

(II)

in which m, n and X have the abovementioned meaning, are hydrogenated.

7. Process according to Claim 6, characterised in that the hydrogenation is carried out catalytically at room temperature and under pressures of 1 to 100 atmospheres.

8. Medicaments containing at least one compound according to Claims 1-5.

**Revendications**

1. Composés de formule

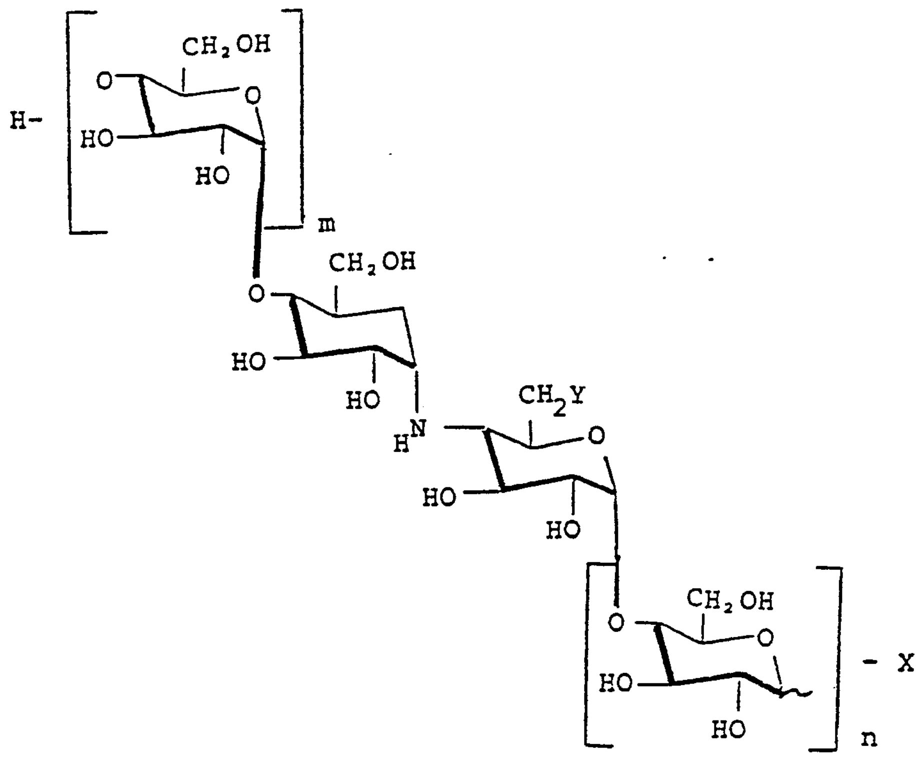

dans laquelle

m et n représentent chacun, indépendamment l'un de l'autre, un nombre entier de 0 à 8, la somme m + n ayant une valeur de 0 à 8,

X représente OH, OR, SH, SR, $NH_2$, NHR ou $NRR_1$, et

R et $R_1$ représentent des groupes alkyle, cycloalkyle, aryle, aralkyle ou hétérocyclyle éventuellement substitués, ou forment ensemble et avec l'atome d'azote auquel ils sont reliés un noyau hétérocyclique, et

Y représente un groupe H ou OH.

2. Composés selon la revendication 1, caractérisés en ce que m et n représentent un nombre de 0 à 3.

3. Composés selon la revendication 1, caractérisés en ce que m = 0 et n = 0 ou 2.

4. Composés selon les revendications 1 à 3, caractérisés en ce que X représente OH.

5. Composés selon les revendications 1 à 4, caractérisés en ce que Y représente H.

6. Procédé de préparation des composés de formule générale I

(Voir figure page suivante)

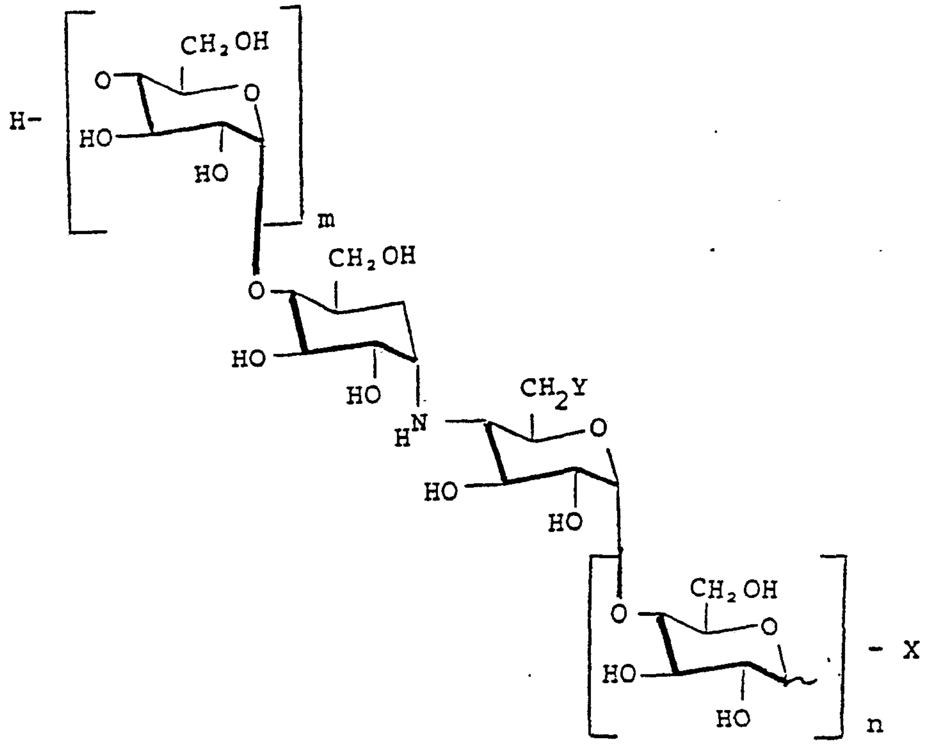

(I)

dans laquelle

m et n représentent chacun, indépendamment l'un de l'autre, un nombre entier de 0 à 8 et la somme m + n a une valeur de 0 à 8,

X représente OH, OR, SH, SR, $NH_2$, NHR ou $NRR_1$,

Y représente H ou OH, et

R et $R_1$ représentent des groupes alkyle, cycloalkyle, aryle, aralkyle ou hétérocyclyle éventuellement substitués ou forment ensemble et avec l'atome d'azote auquel ils sont reliés un noyau hétérocyclique, caractérisé en ce que l'on hydrogène des composés de formule générale II

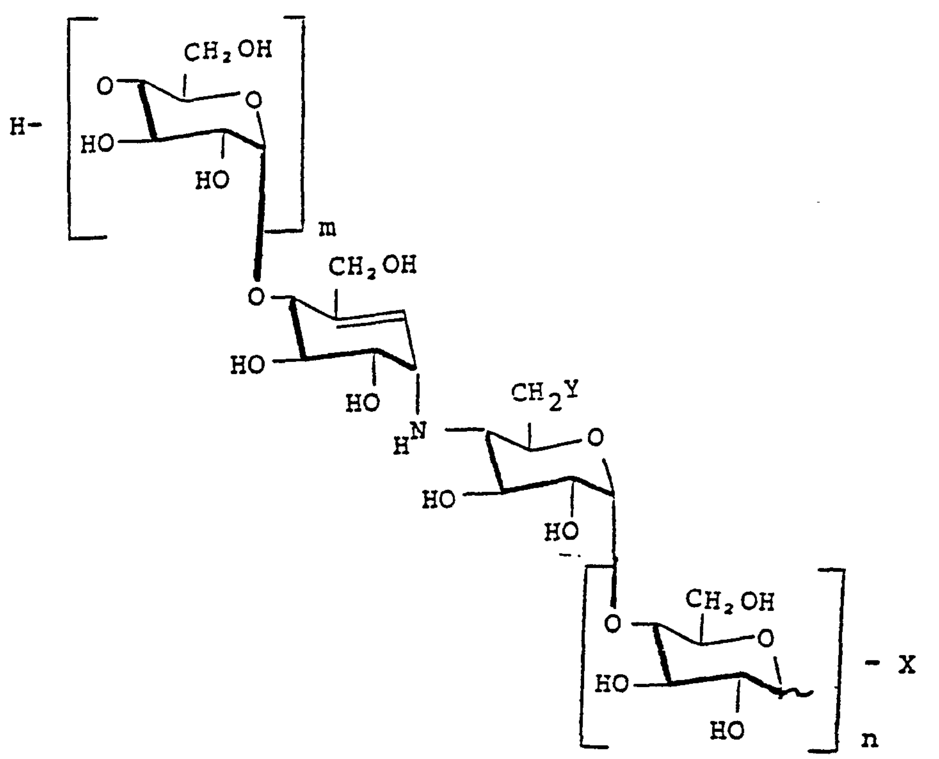

(II)

dans laquelle m, n et X ont les significations indiquées ci-dessus.

7. Procédé selon la revendication 6, caractérisé en ce que l'hydrogénation est une hydrogénation catalytique effectuée à température ambiante et à des pressions de 1 à 100 atmosphères.

8. Médicament contenant au moins un composé selon les revendications 1 à 5.